# EUROPÄISCHE PATENTSCHRIFT

(11) **EP 0 825 949 B1**
(45) Veröffentlichungstag und Bekanntmachung des Hinweises auf die Patenterteilung: **16.04.2003**
(21) Anmeldenummer: 97907025.7
(22) Anmeldetag: 29.01.1997
(51) Int. Cl.: B65B 3/00, B65G 47/90

(54) **VORRICHTUNG ZUM HANDHABEN VON IN EINEM VERPACKUNGSBEHALTER ANGEORDNETEN GEGENSTANDEN**
DEVICE FOR HANDLING OBJECTS IN A PACKAGING CONTAINER
DISPOSITIF POUR MANIPULER DES OBJETS PLACES DANS UN RECIPIENT D'EMBALLAGE

(30) Priorität: 06.02.1996 DE 19604100
(43) Veröffentlichungstag der Anmeldung: 04.03.1998
(73) Patentinhaber: ROBERT BOSCH GMBH, 70442 Stuttgart (DE)
(72) Erfinder: GÖTZELMANN, Bernd, D-74585 Rot am See (DE)
(86) Internationale Anmeldenummer: DE9700156
(87) Internationale Veröffentlichungsnummer: WO97029015

(56) Entgegenhaltungen:
- WO-A-94/04415
- DE-C- 4 332 095
- US-A- 3 722 719
- US-A- 4 456 115
- US-A- 4 478 094
- US-A- 5 377 815

## Beschreibung

Die Erfindung betrifft eine Vorrichtung zum Handhaben von in einem nach oben offenen Behälter angeordneten, befüllbaren, rohrförmigen Gegenständen. Aus der EP 0 257 230 A1 ist eine Vorrichtung bekannt geworden, die eine bewegliche Übernahmeeinheit mit Aufnahmen aufweist, in denen Gegenstände durch Unterdruck festgehalten werden. Durch die Verwendung von Unterdruck kann nur eine beschränkte Haltekraft auf den Gegenstand übertragen werden. Weiterhin müssen die Aufnahmen sehr genau der Kontur der Gegenstände angepasst werden, damit an den Saugöffnungen zum Gegenstand keine Leckluftverluste auftreten, die die Haltekraft zusätzlich reduzieren. Ferner ist es bekannt, beispielweise Spritzenkörper in sogenannten Trays zu bevorraten. Bei einem Tray handelt es sich um einen Behälter, der eine Vielzahl von Stegen aufweist, zwischen denen jeweils ein Spritzenkörper form- und/oder kraftschlüssig angeordnet ist. Zum Befüllen der Spritzenkörper, beispielsweise mit einem Medikament, müssen die Spritzenkörper aus dem Tray entnommen, zuerst einer Reinigungs- und Sterilisiereinrichtung, und anschließend einer Füll- und Verschließeinrichtung zugeführt werden. Anschließend werden die befüllten und verschlossenen Spritzenkörper wieder in das Tray eingelegt. Bei einem bekannten Verfahren zum Herausnehmen der Spritzenkörper aus dem Tray wird das Tray einer Station zugeführt, in der ein keilförmiger Trennkörper angeordnet ist. Dieser Trennkörper greift in einen Zwischenraum zwischen den Spritzenkörpern und der Wand des Trays ein. Durch die Relativbewegung des Trays zum keilförmigen Körper werden die Spritzenkörper nacheinander aus den Stegen herausgedrückt, und in eine Fördereinrichtung übergeben. Das beschriebene Verfahren beansprucht sowohl die Spritzenkörper als auch das Tray mechanisch stark, so daß Beschädigungen an den Spritzenkörpern nicht ausgeschlossen werden können. Weiterhin ist die bekannte Station beispielsweise durch die von der Geometrie der Spritzenkörper und der Trays abhängigen Führungen relativ aufwendig aufgebaut und verursacht bei einem Formatwechsel einen hohen Handhabungsaufwand. Zum Wiedereinführen der befüllten Spritzenkörper in ein Tray ist es bekannt, das leere Tray in geringem Abstand synchron so an einem Förderstem für die Spritzenkörper vorbeizuführen, daß die Spritzenkörper vom Förderstern zwischen die jeweiligen Stege des Trays eingedrückt werden. Anschließend wird das befüllte Tray mittels einer zusätzlichen Fördereinrichtung beispielsweise einem Magazinierspeicher für die Trays zugeführt. Durch die zusätzliche Fördereinrichtung ist ein relativ hoher Bauaufwand für die Fördereinrichtung erforderlich. Auch ist bei einem Formatwechsel ein aufwendiger Umbau der Vorrichtung notwendig. Ferner ist es aus der CH-PS 462 711 bekannt, Ampullen zwischen Zungen eines im wesentlichen streifenförmigen Verpackungsbandes einzulegen, in dem das Verpackungsband über eine Trommel läuft, so daß sich der Abstand der Zungen beim Abrollen vergrößert. Ein derartiges Verfahren ist bei Trays jedoch kaum möglich, da ein aus Kunststoff bestehende Tray eine höhere Biegesteifigkeit aufweist als das Verpackungsband. Aufgabe der Erfindung ist es, eine Vorrichtung zum Handhaben von in einem nach oben offenen Behälter angeordneten, befüllbaren, rohrförmigen Gegenständen derart auszubilden, daß bei einem Behälter mit form- und/oder kraftschlüssiger Halterung der Gegenstände die Gegenstände ohne weitere Hilfsmittel auf einfache, rasche Weise sicher und schonend entnommen werden können, wobei auch der Behälter nicht übermäßig beansprucht werden soll. Diese Aufgabe wird durch die kennzeichnenden Merkmale des Anspruchs 1 gelöst. Weitere Vorteile und vorteilhafte Weiterbildungen der erfindungsgemäßen Vorrichtung ergeben sich aus den Unteransprüchen und der Beschreibung. In einer bevorzugten Ausführungsform der Erfindung ist die Vorrichtung mit einem Arm eines Handhabungsroboters verbunden. Dadurch können die Behälter vor und nach dem Befüllen mit den Spritzenkörpern direkt entmagaziniert oder magaziniert bzw. gestapelt werden. Die Ankoppelung der Vorrichtung an einen Handhabungsroboter hat darüber hinaus den besonderen Vorteil, daß sämtliche bei bekannten Vorrichtungen notwendigen, von der Behältergeometrie abhängigen Führungen und Antriebsglieder entfallen können, und daß jeder beliebige Punkt innerhalb der Reichweite des Handhabungsroboters angesteuert werden kann. Dadurch wird eine große Flexibilität der Vorrichtung erzielt.

Besonders vorteilhaft ist es, die Übernahmeeinheit mit einer Patrize zu koppeln. Dadurch ist zusätzlich ein sowohl für die Spritzenkörper als auch für das Tray schonendes Entnehmen und Wiedereinführen der Spritzenkörper aus bzw. in das Tray möglich.

In einer weiteren bevorzugten Ausführungsform der Erfindung ist die Vorrichtung mit einem Arm eines Handhabungsroboters verbunden. Dadurch können die Trays bzw. das SCF-System vor und nach dem Befüllen mit den Spritzenkörpern direkt entmagaziniert oder magaziniert bzw. gestapelt werden. Die Ankopplung der Vorrichtung an einen Handhabungsroboter hat darüber hinaus den besonderen Vorteil, daß sämtliche bei bekannten Vorrichtungen notwendigen, geometrieabhängigen Führungen und Antriebsglieder entfallen können, und daß jeder beliebige Punkt innerhalb der Reichweite des Handhabungsroboters angesteuert werden kann. Dadurch wird eine große Flexibilität der Vorrichtung erzielt.

### Zeichnung

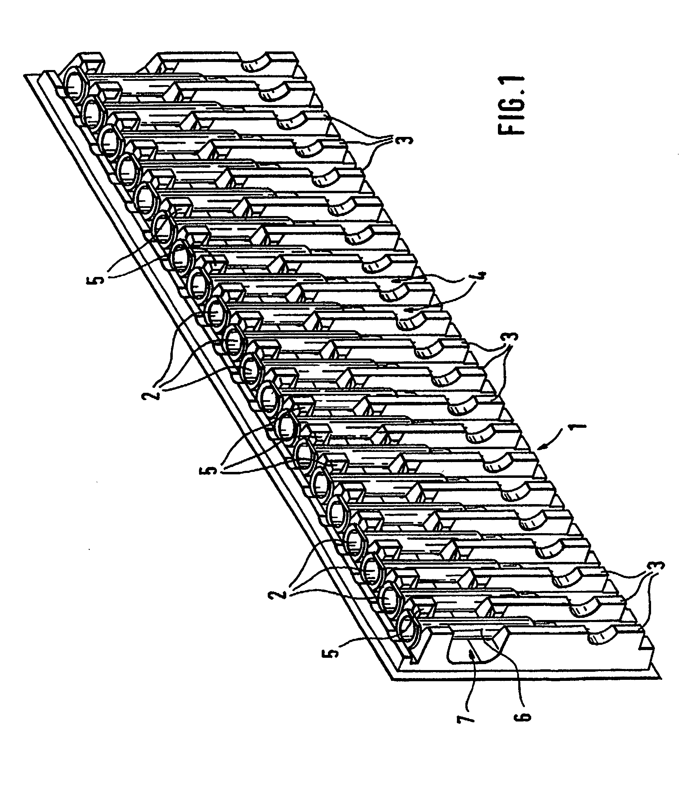

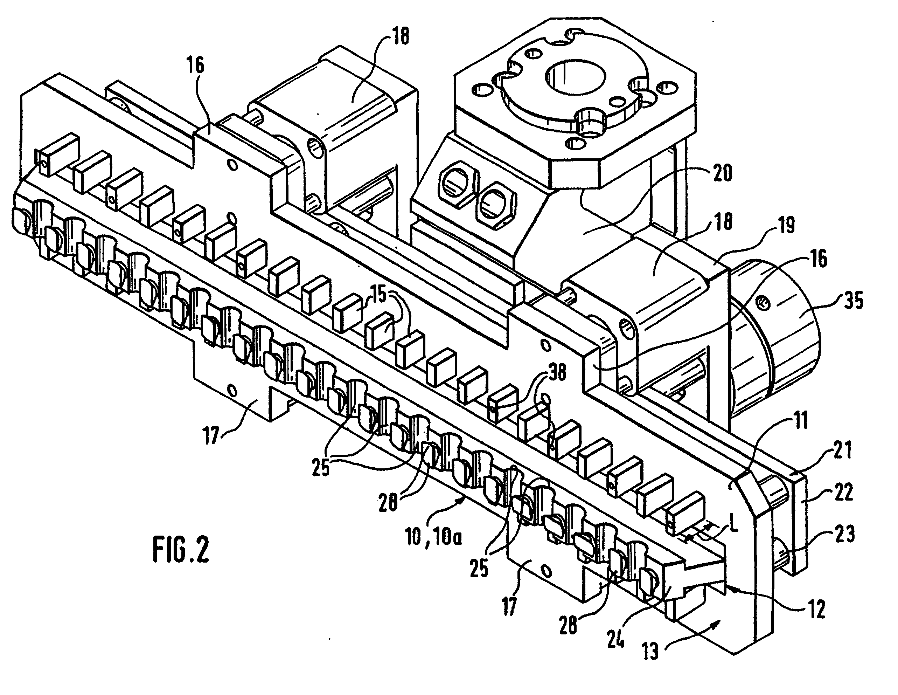

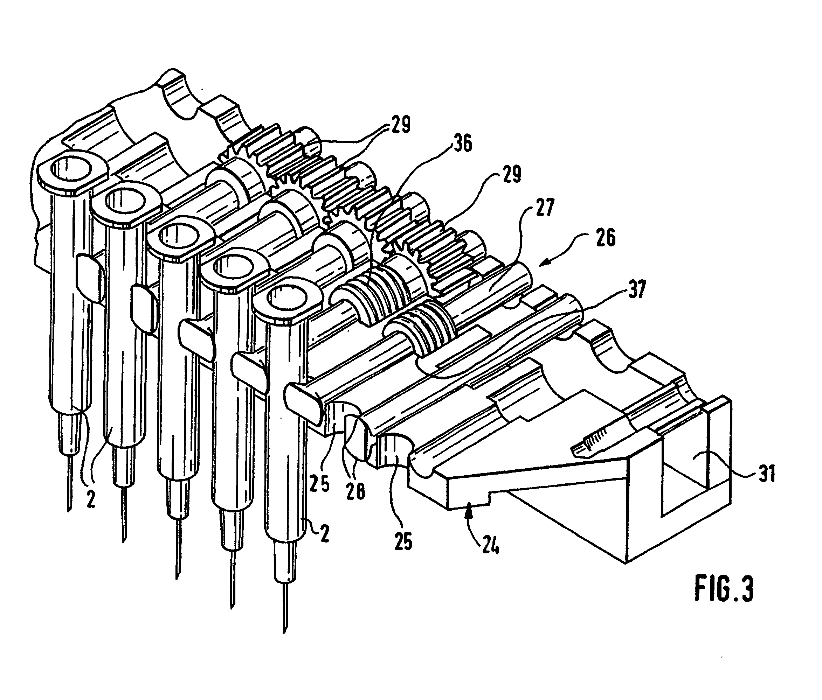

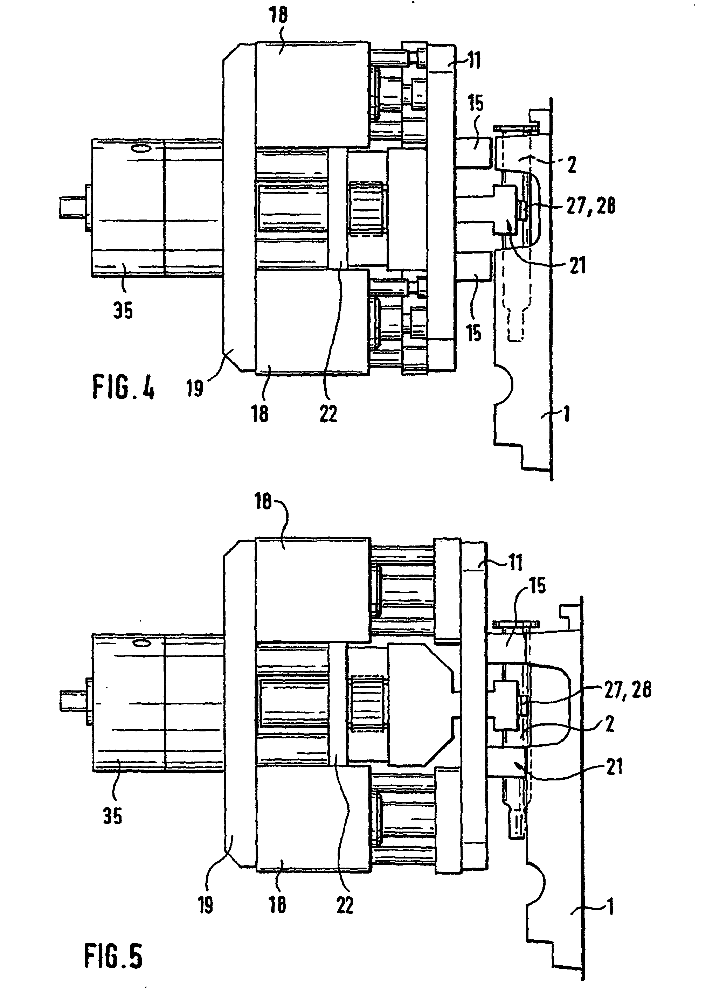

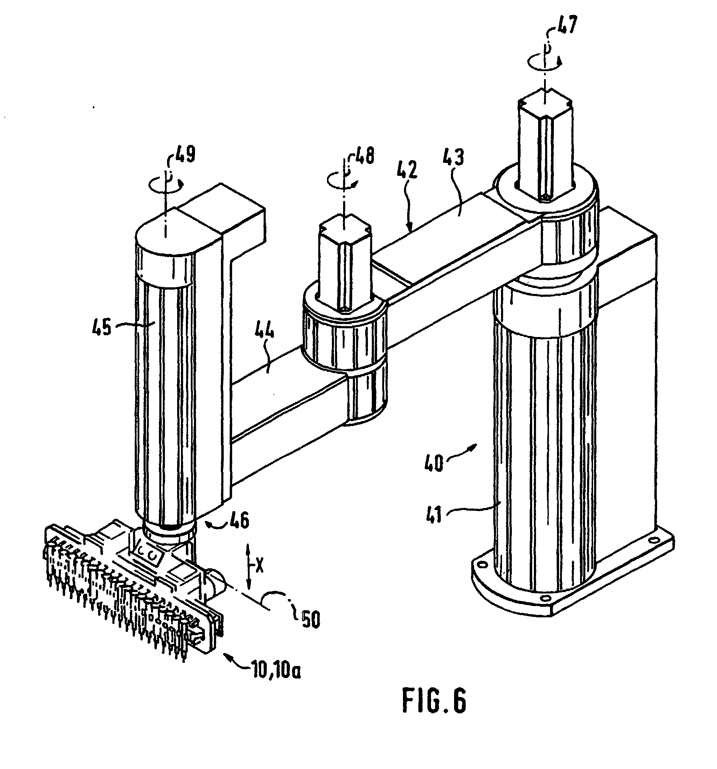

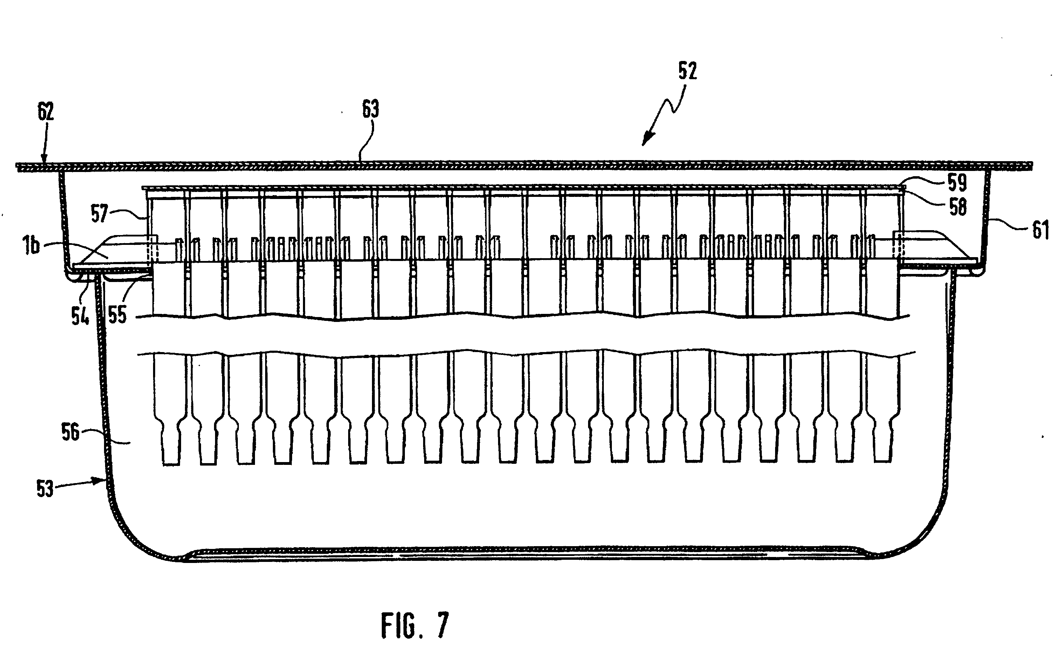

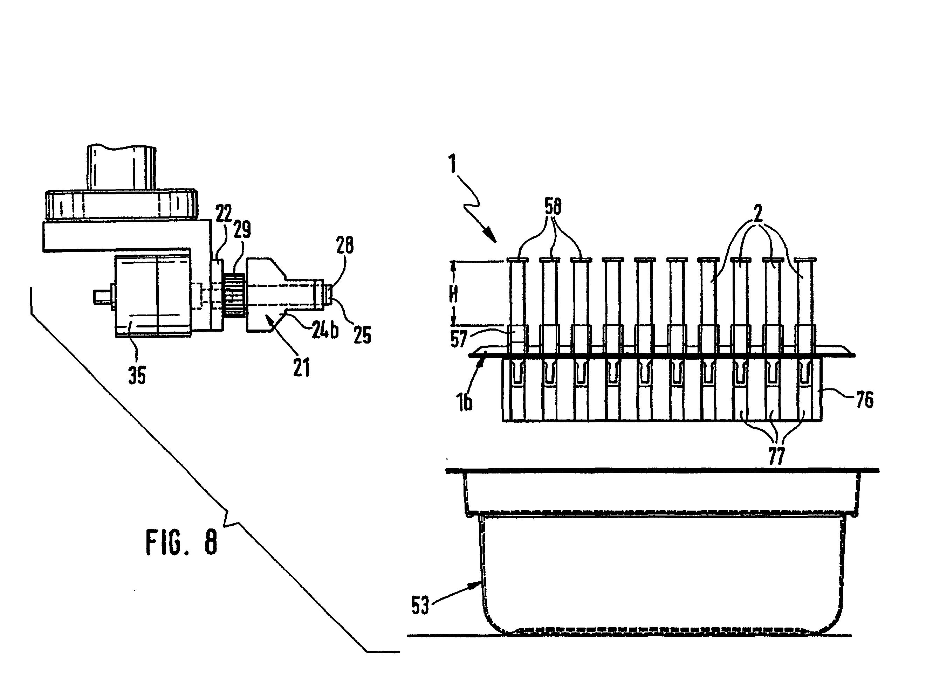

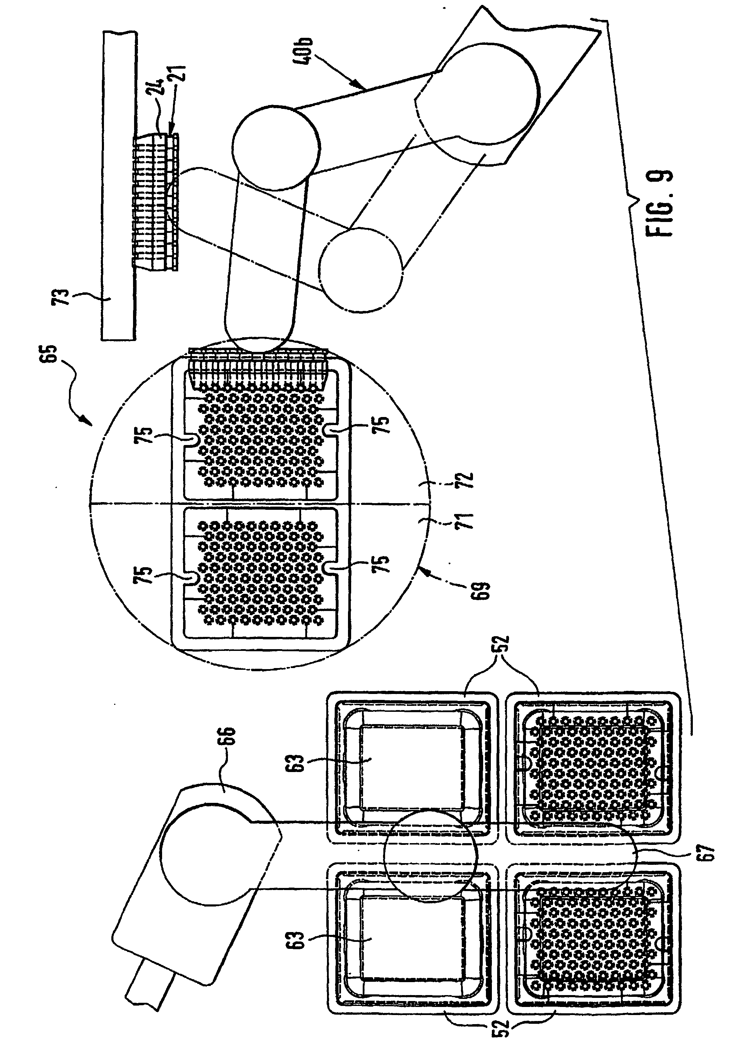

Zwei Ausführungsbeispiele der Erfindung sind in der Zeichnung dargestellt und werden in der nachfolgenden Beschreibung näher erläutert. Es zeigen Figur 1 ein mit Spritzenkörpern befülltes Tray in perspektivischer Ansicht, Figur 2 eine Vorrichtung zum Handhaben von in einem Verpackungsbehälter angeordneten Gegenständen in perspektivischer Ansicht, Figur 3 einen Teil der Vorrichtung nach Figur 2 in perspektivischer Ansicht, die Figuren 4 und 5 die Vorrichtung nach Figur 2 während zweier Betriebsphasen in Seitenansicht, Figur 6 ein Handhabungssystem für Gegenstände in perspektivischer Ansicht, Figur 7 ein verschlossenes SCF-System in einer Seitenansicht und teilweise im Schnitt, Figur 8 die Handhabung des SCF-Systems nach Figur 7 in Seitenansicht und Figur 9 den Eingangsbereich eine Verpackungsanlage für SCF-Systeme in Draufsicht.

### Beschreibung der Ausführungsbeispiele

Die Vorrichtung 10 dient beim ersten Ausführungsbeispiel der Erfindung zum Handhaben von in sogenannten Trays 1 angeordneten Spritzenkörpern 2 (Figur 1). Die an sich bekannten, aus Kunststoff hergestellten, tiefgezogenen Trays 1 haben Stege 3, zwischen denen jeweils die Spritzenkörper 2 in Aussparungen 4 parallelachsig angeordnet sind. Die Spritzenkörper 2 werden mittels Formschluß durch an den Stegen 3 angeformten Traynoppen 5 in dem Tray 1 festgehalten. In dem Bereich, in dem die Spritzenkörper 2 ihren hohlzylindrischen Mittelabschnitt 6 haben, sind in den Stegen 3 Längsaussparungen 7 ausgebildet.

In den oben beschriebenen Trays 1 sind in der Regel eine Vielzahl von Spritzenkörpern 2, beispielsweise zwanzig Stück, angeordnet. Die Trays 1 werden stapelweise an den Verpacker geliefert, wo mit Hilfe der Vorrichtung 10 die Spritzenkörper 2 aus den Trays 1 entnommen werden. Dann werden die Spritzenkörper 2 zuerst einer nicht dargestellten Reinigungs- und Sterilisiereinrichtung, und anschließend einer Befüll- und Verschließeinrichtung für die Spritzenkörper 2 zugeführt. Nach dem Befüllen der Spritzenkörper 2 und dem Verschließen mit jeweils einem Verschlußstopfen werden die Spritzenkörper 2 wieder in die Trays 1 eingebracht.

Die Vorrichtung 10 hat eine im wesentlichen rechteckige, plattenförmige Patrize 11 mit einem darin mittig ausgebildeten Längsschlitz 12 (Figur 2). Ober- und unterhalb des Längsschlitzes 12 sind auf der Vorderseite 13 der Patritze 11 klotzförmige Abdrückstege 15 für einen Tray 1 befestigt. Die Anordnung der Abdrückstege 15 auf der Patritze 11 ist derart, daß diese in Deckung mit den Stegen 3 des Trays 1 angeordnet sind, wobei sich jeweils ein Abdrücksteg 15 ober- und unterhalb einer Längsaussparung 7 eines Steges 3 befindet. Weiterhin ist die Länge L der Abdrückstege 15 unterschiedlich, wobei die an den seitlichen Randbereichen der Patrize 11 angeordneten Abdrückstege 15 eine größere Länge L aufweisen als die im Mittelbereich der Patrize 11 angeordneten Abdrückstege 15. Dadurch ergibt sich in einer Draufsicht auf die Patrize 11 eine Kreis- bzw. Bogenkontur der Abdrückstege 15.

An der oberen bzw. unteren Stirnfläche der Patrize 11 sind jeweils zwei Fortsätze 16, 17 angeformt. Mit jeweils einem Fortsatzpaar 16, 17 ist auf der den Abdrückstegen 15 gegenüberliegenden Seite der Patrize 11 jeweils eine pneumatisch betriebene Hubeinheit 18 verbunden, wobei die beiden Hubeinheiten 18 synchron betrieben werden. Die beiden Hubeinheiten 18 sind mit einem gemeinsamen Grundträger 19 verbunden, der seinerseits an eine zwischen den beiden Hubeinheiten 18 angeordnete Schwenk- und Dreheinrichtung 20 angekoppelt ist.

An der Schwenk- und Dreheinrichtung 20 ist eine Übernahmeeinheit 21 für die Spritzenkörper 2 befestigt. Die Übernahmeeinheit 21 besteht aus einer mit dem Grundträger 19 verbundenen Platte 22, die mittels Distanzhülsen 23 mit einem eine horizontale Trennebene aufweisenden Spritzenkörperträger 24 verbunden ist. Die dem Grundträger 19 zugewandte Platte 22 befindet sich unabhängig von der Hubbewegung der Patrize 11 stets zwischen dem Grundträger 19 und der Patrize 11. Der Spritzenkörperträger 24 durchdringt den Längsschlitz 12 der Patrize 11.

Wie Figur 3 zeigt, weist der Spritzenkörperträger 24 im Bereich zwischen der oberen und der unteren Reihe der Abdrückstege 15, d.h. also im Bereich des Längsschlitzes 12, den Spritzenkörpern 2 angepaßte, im Ausführungsbeispiel halbkreisförmige Aussparungen 25 auf, die in etwa die halbe Querschnittsfläche der Spritzenkörper 2 überdecken. Zwischen den Aussparungen 25 sind in Höhe der Aussparungen 25 drehbewegliche Schließelemente 26 angeordnet.

Die Schließelemente 26 bestehen aus jeweils einem im wesentlichen zylindrisch geformten Stift 27, an dessen einer, den Aussparungen 25 zugewandten Stirnseite zwei Haltenasen 28 für die Spritzenkörper 2 angeformt sind. In der einen Endposition der Stifte 27 sind die Haltenasen 28 mit den Längsachsen der Spritzenkörper 2 ausgerichtet, so daß die Aussparungen 25 vollständig für die Spritzenkörper 2 freigegeben sind. In der anderen, um neunzig Grad gedrehten Endlage der Stifte 27 verdecken die Haltenasen 28 die Öffnungen der Aussparungen 25 zumindest teilweise, so daß ein in einer Aussparung 25 angeordneter Spritzenkörper 2 durch die Aussparung 25 und die zugeordneten Haltenasen 28 zweier Stifte 27 formschlüssig umfasst ist.

Auf der den Haltenasen 28 gegenüberliegenden Seite ist am Stift 27 ein Zahnrad 29 drehfest befestigt, das in einer im Spritzenkörperträger 24 ausgebildeten Aussparung 31 angeordnet ist. Die einzelnen Zahnräder 29 der Stifte 27 kämmen ineinander, so daß bei einer Drehung eines Stiftes 27 die anderen Stifte 27 zwangsläufig synchron mitgedreht werden. Um die Drehbewegung der Stifte 27 zwischen den beiden Endpositionen zu ermöglichen, ist wenigstens einer der Stifte 27 über sein Zahnrad 29 hinaus verlägert, und mit einem im Bereich des Grundträgers 19 angeordneten Stellmotors 35 gekoppelt.

Zwischen den Haltenasen 28 und dem Zahnrad 29 ist jedem Stift 27 eine Druckfeder 36 zugeordnet, die sich einerseits gegen den Stift 27, und andererseits gegen einen Absatz 37 im Spritzenkörperträger 24 abstützt, und die den Stift 27, und somit auch die Haltenasen 28, in Richtung zur Aussparung 25 drückt.

Die oben beschriebene Vorrichtung 10 ist zum Herausnehmen der Spritzenkörper 2 aus einem Tray 1 geeignet. Das Wiedereinführen von (befüllten) Spritzenkörpern 2 in leere Trays 1 erfolgt mittels einer modifizierten Vorrichtung 10a. Diese Vorrichtung 10a (Figur 2) unterscheidet sich von der Vorrichtung 10 dadurch, daß in mehreren Abdrückstegen 15, beispielsweise in acht Abdrückstegen 15, Saugbohrungen 38 ausgebildet sind, die in den den Stegen 3 des Trays 1 zugewandten Stirnflächen der Abdrückstege 15 münden, und die mit einer nicht dargestellten Vakuumquelle verbunden sind.

Sowohl die Vorrichtung 10, als auch die Vorrichtung 10a ist mit ihrem jeweiligen Grundträger 20 an einen Handhabungsroboter 40 ankoppelbar (Figur 6). Der Handhabungsroboter 40 weist eine Stütze 41 auf, von der ein Arm 42 mit zwei gelenkig miteinander verbundenen Abschnitten 43, 44 ausgeht, an dessen einem Abschnitt 44 eine Hubeinheit 45 angeschlossen ist. Am unteren Ende 46 der Hubeinheit 45 ist die Vorrichtung 10, 10a mittels der Schwenk- und Dreheinrichtung 20 angeschlossen. Der Handhabungsroboter 40 weist insgesamt fünf Freiheitsgrade auf: Der Arm 42 ist in der ersten senkrechten Achse 47 in der Stütze 41 drehbar. Weiterhin sind die beiden Abschnitte 43, 44 in der zweiten senkrechten Achse 48 drehbar gelagert. Die dritte senkrechte Achse 49 wird durch die Drehbeweglichkeit der Vorrichtung 10, 10a in der Schwenk- und Rotationseinrichtung 20 realisiert. Weiterhin ist die Vorrichtung 10, 10a durch die Hubeinheit 45 in Richtung X heb- bzw. senkbar, sowie durch die Schwenk- und Dreheinrichtung 20 um eine horizontal angeordnete Achse 50 um mindestens 90 Grad schwenkbar.

Die oben beschriebene Vorrichtung 10, 10a zum Handhaben von Spritzenkörpern 2 arbeitet wie folgt: Zum Entnehmen der Spritzenkörper 2 aus einem beispielweise in einem Stapelmagazin bereitgestellten Tray 1 ist am Handhabungsroboter 40 die Vorrichtung 10 montiert. Ferner sind die Haltenasen 28 in der einen, die Aussparungen 25 in dem Spritzenkörperträger 24 freigebenden Position gedreht, sowie die Patrize 11 mittels der Hubeinheiten 18 in die vom Grundträger 19 nächste Position verfahren. In dieser Grundeinstellung der Vorrichtung 10 wird der Handhabungsroboter 40 in Richtung des Trays 1 geschwenkt, wobei es aufgrund der Freiheitsgrade des Handhabungsroboters 40 unerheblich ist, ob das Tray 1 liegend oder stehend angeordnet ist. Voraussetzung ist lediglich, daß die den Spritzenkörpern 2 zugeordnete Vorderseite des Trays 1 frei zugänglich ist. Nachdem die Aussparungen 25 des Spritzenkörperträgers 24 zu den Spritzenkörpern 2 im Tray 1 ausgerichtet sind, wird der Spritzenkörperträger 24 in das Tray 1 hineinverfahren, bis die Spritzenkörper 2 in Anlage mit den Aussparungen 25 des Spritzenkörperträgers 24 gelangen. In dieser Position werden die Stifte 27 mittels des Stellmotors 35 um neunzig Grad verdreht, so das die Haltenasen 28 der Stifte 27 die Spritzenkörper 2 umfassen (Figur 4). Da die Druckfedern 36 die Haltenasen 28 in Richtung der Aussparungen 25 drücken, findet ein form- und kraftschlüssiges Halten der Spritzenkörper 2 in dem Spritzenkörperträger 24 statt.

Nun transportiert der Handhabungsroboter 40 die Vorrichtung 10 samt Tray 1 und Spritzenkörper 2 zu einer Ablege- bzw. Sammelstelle für leere Trays 1. Dort findet die Trennung der Spritzenkörper 2 vom Tray 1 statt.

Um die Spritzenkörper 2 aus dem Tray 1 zu entnehmen wird die Patrize 11 mittels der Hubeinheiten 18 in Richtung der Aussparungen 25 des Spritzenkörperträgers 24 verfahren. Dadurch gelangen die Abdrückstege 15 nach und nach in Kontakt zu den Stegen 3 des Trays 1 und drücken das Tray 1 von den Spritzenkörpern 2 weg, bis diese von den Stegen 3 freigegeben sind (Figur 5). Das Freigeben der einzelnen Spritzenkörper 2 erfolgt entsprechend der unterschiedlichen Längen L der Abdrückstege 18 nach und nach, von den seitlichen Randzonen der Patrize 11 beginnend, hin zu deren Mittelbereich.

Nachdem die Spritzenkörper 2 aus dem Tray 1 entnommen sind, wird der Handhabungsroboter 40 mit den in dem Spritzenkörperträger 24 angeordneten Spritzenkörpern 2 weiterverfahren, und übergibt die Spritzenkörper 2 (nach Freigeben der Spritzenkörper 2 durch Rückdrehen der Stifte 27 in ihre ursprüngliche, die Aussparungen 25 freigebende Position) in eine nicht dargestellte, an sich bekannte kontiniuerlich oder taktweise arbeitende Fördereinrichtung. Mittels der Fördereinrichtung werden die Spritzenkörper 2 zuerst einer Reinigungs- und Sterilisiereinrichtung, und anschließend einer Befüll- und Verschließeinrichtung für die Spritzenkörper 2 zugeführt, aus der sie anschließend wieder ausgeschleust, und in den Bereich eines zweiten Handhabungsroboters 40a zugefördert werden. An diesem zweiten Handhabungsroboter 40a ist im dargestellten Ausführungsbeispiel die Vorrichtung 10a montiert. Das Übergeben der befüllten und mittels Verschlußstopfen verschlossenen Spritzenkörper 2 zwischen die Stege 3 eines leeren Trays 1 erfolgt dadurch, daß die Spritzenkörper 2 in einem ersten Arbeitsschritt von der Fördereinrichtung in die Aussparungen 25 des Spritzenkörperträgers 24 eingebracht werden. Sobald die Spritzenkörper 2 form- und kraftschlüssig in dem Spritzenkörperträger 24 angeordnet sind (nach entsprechendem Drehen der Stifte 27 in die die Aussparungen 25 überdeckende Endposition) schwenkt der Handhabungsroboter 40a die Vorrichtung 10a in den Bereich eines bereitgestellten, leeren Trays 1. Nach dem Ausrichten der Abdrückstege 15 mit den Stegen 3 des Trays 1 werden die Abdrückstege 15 in Kontakt mit den zugeordneten Stegen 3 des Trays 1 gebracht. Durch Einschalten der Vakuumquelle werden über die Saugbohrungen 38 die Stege 3, und somit das Tray 1 angesaugt, und gegenüber dem Spritzenkörperträger 24 positioniert.

Anschließend schwenkt der Handhabungsroboter 40a die Vorrichtung 10a samt Tray 1 in den Bereich beispielsweise einer ortsfesten, bevorzugt geringfügig schräg zur Horizontalen angeordneten, gefedert gelagerten, ebenen Platte für das Tray 1. Diese Platte bildet ein Gegenlager für die Patrize 11 für das nachfolgende Einschieben der Spritzenkörper 2 in das Tray 1.

Das Einschieben der Spritzenkörper 2 zwischen die Stege 3 des Trays 1 erfolgt dadurch, daß (nach Schwenken der Schwenk- und Dreheinrichtung 20 um die horizontale Achse 50) zum Ablegen des Trays 1 auf der Platte das Vakuum, mit der das Tray 1 gehalten wurde, abschaltet, und die Patrize 11 zurückverfahren, d.h. in Richtung zum Grundträger 19 bewegt wird. Gleichzeitig wird von der Hubeinheit 45 die Vorrichtung 10a in Richtung gegen das leicht schräg liegende Tray 1 verfahren, so daß die Spritzenkörper 2 nach und nach zwischen die Traynoppen 5 der Stege 3 eingedrückt werden.

Nach dem Einschieben aller Spritzenkörper 2 zwischen die Stege 3 des Trays 1, und dem eventuellen Ablegen des nunmehr befüllten Trays 1 an einem anderen Ort durch den Handhabungsroboter 40a, werden die Stifte 27 mittels des Stellmotors 35 wieder in ihre die Aussparungen 25 freigebende Endlage verdreht, so daß das Tray 1 frei ist.

Anstelle des oben beschriebenen Wiedereinschiebens der Spritzenkörper 2 in das leere Tray 1 mittels der Vorrichtung 10a und der als Gegenlager wirkenden ebenen Platte ist das Wiedereinschieben der Spritzenkörper 2 auch mit der vom Aufbau her gesehen einfacheren Vorrichtung 10 denkbar. In diesem Fall werden die Spritzenkörper 2 nach dem Ausrichten mit dem leeren Tray 1 zwischen die unterhalb der Spritzenkörper 2 (flachliegendes Tray 1) angeordneten Stege 3 eingedrückt. Dies erfolgt dadurch, daß der Vertikalhub des Handhabungsroboters 40 ausgenützt wird, um die Spritenkörper 2 mit einer entsprechend hohen Geschwindigkeit zwischen die Stege 3 des Trays 1 einzupressen.

Ergänzend wird darauf hingewiesen, daß aufgrund der üblicherweise kontinuierlich arbeitenden Reinigungs- und Sterilisiereinrichtung, sowie der Befüll- und Verschließeinrichtung für die Spritzenkörper 2 ein erster Handhabungsroboter 40 mit einer Vorrichtung 10 zum Beschicken der Fördereinrichtung, und ein zweiter Handhabungsroboter 40 oder 40a mit einer Vorrichtung 10 oder 10a zum Entnehmen der befüllten Spritzenkörper 2 aus der Fördereinrichtung erforderlich ist. Bei entsprechend vorhandenen Pufferzonen in der Fördereinrichtung ist es jedoch auch denkbar, lediglich einen Handhabungsroboter 40, 40a mit einer Vorrichtung 10, 10a einzusetzen.

Eine derart modifizierte Anordnung kann beispielsweise derart betrieben werden, daß mittels der Vorrichtung 10, 10a abwechselnd ein Tray 1 entleert, und anschließend ein leeres Tray 1 mit derselben Vorrichtung 10, 10a mit Spritzenkörpern 2 befüllt wird.

Bei dem in den Figuren 7 bis 9 dargestellten zweiten Ausführungsbeispiel der Erfindung dient die Vorrichtung 10b zum Handhaben von Spritzenkörpern 2, die in einem als Verpackungsbehälter dienenden sogenannten SCF-System 52 angeordnet sind. Das SCP-System 52 weist einen wannenförmigen, vorzugsweise aus Kunststoff bestehenden Behälter 53 auf. Im oberen Bereich des Behälters 53 ist ein horizontal umlaufender Rand 54 ausgebildet, auf dem ein Tray 1b dichtend aufliegt. Das ebenfalls aus Kunststoff bestehende Tray 1b weist mehrere Reihen neben- und hintereinander angeordneter Durchbrüche 55 für jeweils einen Spritzenkörper 2 auf. Auf der dem sterilen Innenraum 56 des Behälters 53 abgewandten Oberseite des Trays 1b ist jedem Durchbruch 55 eine Spritzenaufnahmehülse 57 zugeordnet, die am Tray 1b angeformt ist. Die Spritzenkörper 2 sind von den Spritzenaufnahmehülsen 57 am ihrem jeweiligen Umfang dichtend bzw. klemmend umfasst. Die Spritzenkörper 2 ragen so weit in den Innenraum 56 des Behälters 53 hinein, daß der an der Oberseite der Spritzenkörper 2 flanschförmig umlaufend ausgebildete Rand 58 auf der Oberseite der Spritzenaufnahmehülsen 57 aufliegt. Somit sind die Außerflächen der beim Spritzenhersteller gereinigten und sterilisierten Spritzenkörper 2 vollständig im Innenraum 56 steril untergebracht. Um die Sterilität auch der Innenflächen der Spritzenkörper 2 zu gewährleisten, ist auf der Oberseite der Ränder 58 der Spritzenkörper 2 ein die Spritzenkörper 2 überdeckendes, steriles Abdeckvlies 59 angeordnet. Das oben beschriebene Tray 1b ist samt Spritzenkörper 2 und Abdeckvlies 59 vollständig in dem Behälter 53 eingebracht und dort luftdicht verschlossen. Dazu hat der Behälter 53 in Höhe der Spritzenaufnahmehülsen 57 einen umlaufenden Wandabschnitt 61, auf dessen Oberseite 62 ein luftdichte Schutzfolie 63 aufgeklebt ist.

Die derart beim Spritzenhersteller gefertigten SCF-Systeme 52 gelangen beim Abfüller in einen in der Figur 9 dargestellten Bereitstellungs- und Übergabebereich 65. Dieser Bereich weist sterile Raumbedingungen, beispielsweise nach der Reinraumklasse 100 auf. In dem Bereitstellungs- und Übergabebereich 65 ist ein an sich bekannter Roboter 66 mit einem Greifer 67 angeordnet, der die bereitgestellten SCF-Systeme 52 öffnet, indem er die Schutzfolien 63 und die Abdeckvliese 59 automatisch entfernt. Nachdem dies erfolgt ist, überführt der Roboter 66 den Behälter 53 an einen Schalttisch 69.

Der um eine vertikale Achse drehbare Schalttisch 69 weist einen Vorratsbereich 71 und einen Bearbeitungsbereich 72 für jeweils einen Behälter 53 auf. In dem Vorratsbereich 71 wird der vom Roboter 66 bereitgestellte Behälter 53 abgestellt, um nach einer Drehung um 180 Grad in den Bearbeitungsbereich 72 zu gelangen. In dem Bearbeitungsbereich 72 werden die Spritzenkörper 2 von einem Handhabungsroboter 40b aus dem Behälter 53 und dem Tray 1b wie nachfolgend noch beschrieben entnommen, und einer schematisch dargestellten Transporteinrichtung 73 übergeben. Dabei ist in der Figur 9 mit getrichelten Linien die Übergabeposition des Handhabungsroboters 40b an die Transporteinrichtung 73 eingezeichnet.

Die bei SCF-Systemen 52 verwendete Vorrichtung 10b zum Handhaben der Spritzenkörper 2 unterscheidet sich von den beim ersten Ausführungsbeispiel der Erfindung verwendeten Vorrichtungen 10, 10a vorzugsweise durch einen vereinfachten Aufbau. Insbesondere können die Schwenk- und Dreheinrichtung 20, die Patrize 11 samt Abdrückstegen 15, die Hubeinheiten 18 sowie der Grundträger 19 entfallen, wie dies in der Figur 8 dargestellt ist.

Zum Entnehmen der Spritzenkörper 2 aus dem Behälter 53 hat der Schalttisch 69 Hubelemente 74, von denen jeweils ein Hubelement 74 in eine an der Oberseite 62 des Wandabschnitts 61 ausgebildete Aussparung 75 des Behälters 53 eingreift, und so das Tray 1b aus dem Behälter 53 heraus hebt. Anschließend wird das Tray 1b auf eine Spritzenmatrize 76 abgesenkt. Die Spritzenmatrize 76 hat für jeden Spritzenkörper 2 eine konisch ausgebildete Zentrierbohrung 77. Nachdem die Spritzenkörper 2 durch Absenken des Tray 1b um ein gewisses Maß in die Zentrierbohrungen 77 eingetaucht sind, werden die Spritzenkörper 2 beim weiteren Absenken des Trays 1b aus dem Tray 1b angehoben, bis deren Rand 58 um ein Maß H über die Spritzenaufnahmehülsen 57 überstehen. In dieser Position der Spritzenkörper 2, bei der diese noch in den Spritzenaufnahmehülsen 57 geführt sind, kann die dem Spritzenkörperträger 24b der Vorrichtung 10b zugewandte Reihe von Spritzenkörpern 2 dem Tray 1b entnommen werden. Dazu fährt der Spritzenkörperträger 24b mit seinen Aussparungen 25b wie in der Figur 9 dargestellt gegen die Spritzenkörper 2, welche nach Drehen der Schließelemente 25b in den Aussparungen 25b fixiert sind. Danach erfolgt in bekannter Weise die Übergabe der Spritzenkörper 2 an die Transporteinrichtung 73. Anschließend werden von der Vorrichtung 10b alle Reihen von Spritzenkörpern 2 aus dem Tray 1b auf die oben beschriebene Art entnommen.

Das Wiedereinführen der Spritzenkörper 2 in ein leeres Tray 1b nach dem Befüllen der Spritzenkörper 2 erfolgt in entsprechend umgekehrter Reihenfolge.

In Ergänzung zu den beiden beschriebenen Ausführungsbeispielen ist es auch denkbar, die erfindungsgemäße Vorrichtung 10, 10a, 10b auch auf andere Gegenstände bzw. Verpackungen anzuwenden. Jedenfalls ist die Anwendung nicht auf in Trays 1, 1b angeordnete Spritzenkörper 2 beschränkt, sondern kann nach entsprechender Modifikation auch auf andere Anwendungsfälle, insbesondere auf in enger Teilung nebeneinander angeordneter Vials, Carpulen, Ampullen und andere zylindrische Objekte übertragen werden.

## Patentansprüche

1. Vorrichtung zum Handhaben von in einem nach oben offenen Behälter angeordneten, befüllbaren, rohrförmigen Gegenständen, insbesondere Spritzenkörper oder Ampullen,
wobei die Gegenstände (Spritzenkörper 2) lösbar zwischen elastischen Stegen (3) des Behälters (Tray 1) form- und/oder kraftschlüssig gehalten sind,
mit einer beweglichen Übernahmeeinheit (21), die Aussparungen (25) für die Aufnahme der Gegenstände (2) aufweist, wobei die Anordnung der Aussparungen (25) der durch die Stege (3) gebildeten Anordnung beim Behälter (1) entspricht, und die bewegliche Halteelemente besitzt, die in Schließstellung die in den Aussparungen (25) aufgenommenen Gegenstände (2) klemmend hintergreifen und fixieren, **gekennzeichnet durch** eine Patrize (11), die beim Erfassen der Gegenstände (2) **durch** die Übernahmeeinheit (21) gegen die Stege (3) des Behälters (1) anlegbar ist, so daß **durch** eine nachfolgende gegenläufige Bewegung zwischen der Übernahmeeinheit (21) und der Patrize (11) die Gegenstände (2) aus ihrer **durch** die Stege (3) gebildeten Halterung gelöst werden können.

2. Vorrichtung nach Anspruch 1, **dadurch gekennzeichnet, daß** die Halteelemente in Form von Stiften (27) mit Vorsprüngen (28) zum Hintergreifen und Fixieren der Gegenstände (2) ausgebildet sind, und daß die Stifte (27) innerhalb der Übernahmeeinheit (21) angeordnet sind.

3. Vorrichtung nach Anspruch 1 oder 2, **dadurch gekennzeichnet, daß** die Übernahmeeinheit (21) aus einer Platte (22) besteht, die mittels Distanzelementen (23) mit einem Spritzenkörperträger (24) verbunden ist, in dem die Aussparungen (25) ausgebildet sind.

4. Vorrichtung nach einem der Ansprüche 1 bis 3, **dadurch gekennzeichnet, daß** an der im wesentlichen plattenförmig ausgebildeten Patrize (11) auf der dem Behälter (1) zugewandten Seite in Deckung mit den Haltestegen (3) des Behälters (1) mehrere Distanzkörper (15) angeordnet sind.

5. Vorrichtung nach Anspruch 4, **dadurch gekennzeichnet, daß** die Distanzkörper (15) unterschiedliche Längen (L) aufweisen.

6. Vorrichtung nach Anspruch 5, **dadurch gekennzeichnet, daß** die in den seitlichen Randbereichen der Patrize (11) angeordneten Distanzkörper (15) eine größere Länge (L) aufweisen als die im Mittelbereich der Patrize (11) angeordneten Distanzkörper (15), so daß sich in einer Draufsicht auf die Patrize (11) eine bogenförmige Kontur der Distanzkörper (15) ergibt.

7. Vorrichtung nach einem der Ansprüche 4 bis 6, **dadurch gekennzeichnet, daß** in den Distanzkörpern (15) jeweils wenigstens eine Saugbohrung (38) ausgebildet ist, die mit einer Vakuumquelle verbunden sind.

8. Vorrichtung nach einem der Ansprüche 1 bis 7, **dadurch gekennzeichnet, daß** die Patrize (11) eine Aussparung (12) aufweist, durch die die Übernahmeeinheit (21) hindurchragt.

9. Vorrichtung nach einem der Ansprüche 1 bis 8, **dadurch gekennzeichnet, daß** die Patrize (11) mit zwei, beidseitig einer Schwenk- und Dreheinrichtung (20) angeordneten, synchron betreibbaren Hubeinheiten (18) zum Bewegen der Patrize (11) relativ zur Übernahmeeinheit (21) gekoppelt ist, wobei die Hubeinheiten (18) auf der der Übernahmeeinheit (21) zugewandten Seite der Patrize (11) angeordnet sind.

10. Vorrichtung nach einem der Ansprüche 1 bis 9, **dadurch gekennzeichnet, daß** die Vorrichtung (10, 10a) mit einem Handhabungsroboter (40, 40a) verbunden ist.

## Claims

1. Device for handling fillable, tubular objects arranged in a container which is open at the top, in particular injection bodies or ampoules, the objects (injection bodies 2) being held releasably between elastic webs (3) of the container (tray 1) with a form and/or force fit,
having a movable transfer unit (21), which has cutouts (25) to accommodate the objects (2), the arrangement of the cutouts (25) corresponding to the arrangement formed by the webs (3) in the container (1), and which has movable holding elements which, in the closed position, engage in a clamping manner behind the objects (2) accommodated in the cutouts (25) and fix them, **characterized by** a male plate (11) which, when the objects (2) are gripped by the transfer unit (21), can be placed against the webs (3) of the container (1), so that, by means of a following opposite movement between the transfer unit (21) and the male plate (11), the objects (2) can be released from their mounting formed by the webs (3).

2. Device according to Claim 1, **characterized in that** the holding elements are constructed in the form of pins (27) having projections (28) to engage behind and fix the objects (2), and **in that** the pins (27) are arranged inside the transfer unit (21).

3. Device according to Claim 1 or 2, **characterized in that** the transfer unit (21) comprises a plate (22) which is connected by means of spacer elements (23) to a syringe body carrier (24), in which the cutouts (25) are formed.

4. Device according to one of Claims 1 to 3, **characterized in that** on the substantially plate-like male plate (11), on the side facing the container (1), a plurality of spacers (15) are arranged to coincide with the holding webs (3) of the container (1).

5. Device according to Claim 4, **characterized in that** the spacers (15) have different lengths (L).

6. Device according to Claim 5, **characterized in that** the spacers (15) arranged in the lateral edge regions of the male plate (11) have a greater length (L) than the spacers (15) arranged in the central region of the male plate (11), so that the result in a plan view of the male plate (11) is a curved contour of the spacers (15).

7. Device according to one of Claims 4 to 6, **characterized in that** in each case at least one suction bore (38) is formed in the spacers (15) and is connected to a vacuum source.

8. Device according to one of Claims 1 to 7, **characterized in that** the male plate (11) has a cutout (12) through which the transfer unit (21) projects.

9. Device according to one of Claims 1 to 8, **characterized in that** the male plate (11) is coupled to two lifting units (18) which are arranged on both sides of a pivoting and turning device (20) and can be operated synchronously to move the male plate (11) relative to the transfer unit (21), the lifting units (18) being arranged on the side of the male plate (11) facing the transfer unit (21).

10. Device according to one of Claims 1 to 9, **characterized in that** the device (10, 10a) is connected to a handling robot (40, 40a).

## Revendications

1. Dispositif pour manipuler des objets tubulaires, pouvant être remplis, disposés dans un récipient ouvert vers le haut, en particulier des corps de seringues ou des ampoules, dans lequel les objets (corps de seringues 2) sont tenus de façon démontable, par sûreté de forme et/ou par action de force, entre des nervures élastiques (3) du récipient (plateau 1),
comprenant une unité de transfert mobile (21) qui présente des évidements (25) pour recevoir les objets (2), la disposition des évidements (25) correspondant à la disposition dans le récipient (1), qui est définie par les nervures (3), et qui possède des éléments de retenue mobiles qui, dans la position de fermeture, accrochent par l'arrière et immobilisent avec serrage les objets (2) logés dans les évidements (25), **caractérisé par**
un poinçon (11) qui, lors de la saisie des objets (2), peut être placé par l'unité de transfert (21), contre les nervures (3) du récipient (1), de sorte que les objets (2) peuvent être dégagés de leur monture formée par les nervures (3) par un mouvement inverse ultérieur entre l'unité de transfert (21) et le poinçon (11).

2. Dispositif selon la revendication 1,
**caractérisé en ce que**
les éléments de retenue sont réalisés sous la forme de tiges (27) présentant des saillies (28) destinées à accrocher les objets (2) par l'arrière et à les immobiliser, et les tiges (27) sont disposées à l'intérieur de l'unité de transfert (21).

3. Dispositif selon la revendication 1 ou 2,
**caractérisé en ce que**
l'unité de transfert (21) est constituée par une plaque (22) qui est reliée, au moyen d'éléments d'entretoises (23), à un support de corps de seringues (24) dans lequel sont formés les évidements (25).

4. Dispositif selon une des revendications 1 à 3,
**caractérisé en ce que**
plusieurs corps d'écartement (15) sont disposés sur le poinçon (11) configuré sensiblement en forme de plaque, sur le côté dirigé vers le récipient (1), en coïncidence avec les nervures de maintien (3) du récipient (1).

5. Dispositif selon la revendication 4,
**caractérisé en ce que**
les corps d'écartement (15) présentent des longueurs (L) différentes.

6. Dispositif selon la revendication 5,
**caractérisé en ce que**
les corps d'écartement (15) disposés dans les régions marginales latérales du poinçon (11) présentent une longueur (L) plus grande que celle des corps d'écartement (15) disposés dans la région centrale du poinçon (11), de sorte que, dans une vue de dessus du poinçon (11), on obtient un contour en forme d'arc des corps d'écartement (15).

7. Dispositif selon une des revendications 4 à 6,
**caractérisé en ce que**
dans chacun des corps d'écartement (15), est formé au moins un passage d'aspiration (38) qui est relié à une source de vide.

8. Dispositif selon une des revendications 1 à 7,
**caractérisé en ce que**
le poinçon (1) présente un évidement (12) à travers lequel fait saillie l'unité de transfert (21).

9. Dispositif selon une des revendications 1 à 8,
**caractérisé en ce que**
le poinçon (11) est accouplé à deux vérins (18), disposés des deux cotés d'un dispositif de basculement et de rotation (20), qui peuvent être mis en action en synchronisme, destinés à déplacer le poinçon (11) par rapport à l'unité de transfert (21), les vérins (18) étant disposés sur le côté du poinçon (11) dirigé vers l'unité de transfert (21).

10. Dispositif selon une des revendications 1 à 9,
**caractérisé en ce que**
le dispositif (10, 10a) est relié à un robot manipulateur (40, 40a).
